# DEMANDE DE BREVET EUROPEEN

(11) **EP 4 494 662 A1**
(43) Date de publication de la demande: **22.01.2025**
(21) Numéro de dépôt: 24315115.6
(22) Date de dépôt: 02.04.2024
(51) Int. Cl.: A61L 2/10, C02F 1/32, C02F 103/00, C02F 103/02

(54) **DISPOSITIF DE TRAITEMENT D'UN FLUIDE PAR RAYONNEMENT ULTRAVIOLET**

(30) Priorité: 02.04.2023 FR 2303242
(71) Demandeur: Enthal.P, 02100 Saint Quentin (FR)
(72) Inventeur: LIAIS, Nicolas, 02100 Saint Quentin (FR)
(74) Mandataire: Cochonneau, Olivier

(57) **Abrégé**

L'invention concerne un dispositif de traitement d'un fluide par rayonnement UV, trouvant notamment une application à la pasteurisation froide dans le domaine alimentaire, ou au traitement de l'eau de tours de refroidissement et de condenseurs évaporatifs.

Le dispositif comprend une chambre 1 fermée par deux couvercles 4, 5, des unités d'émission UV 6, 7 contrôlées par une unité de contrôle 11 et comprenant une gaine 8, 9, comportant un tube 8 comprenant une source d'émission UV 10, et un fourreau 9, par exemple en matériau de type PFE ou FPA, plaqué sur le tube. L'épaisseur du fourreau 9 est uniformément comprise entre 200 µm et 350 µm, de préférence comprise entre 200 µm et 300 µm, encore plus préférentiellement comprise entre 260 µm et 270 µm.

## Description

La présente invention concerne un dispositif de traitement d'un fluide, tel que de l'eau, par rayonnement ultraviolet. Elle trouve par exemple une application à la pasteurisation froide dans le domaine alimentaire, ou au traitement de l'eau de tours de refroidissement et de condenseurs évaporatifs.

Plus généralement, l'invention vise à être utilisée dans les domaines de l'agriculture, des loisirs et de l'industrie, dans toutes les applications de désinfection, d'abattement d'ozone, et de réduction du carbone organique total (COT) dans les liquides, telles que :
- le traitement des eaux pluviales ;
- le traitement des eaux usées pour réutilisation ;
- le traitement contre la légionnelle dans les installations adiabatiques industrielles et les installations de brumisation telles que les tours aéroréfrigérantes ou les brumisateurs agricoles.

L'invention se destine particulièrement au traitement par rayonnement ultraviolet des liquides légèrement opaques, voire chargés, et/ou à toute application de traitement de liquide requérant un haut niveau d'hygiène, telles que les applications dans les domaines de l'industrie agroalimentaire, l'industrie biologique, l'industrie pharmaceutique, et l'industrie microélectronique.

Le rayonnement ultraviolet, hautement énergétique, est très nocif pour les organismes vivants. Il est absorbé par les protéines et les acides nucléiques (ADN et ARN) des organismes (bactéries, virus, protistes, champignons et organismes multi cellulaires), causant le plus souvent des dommages irréversibles à l'échelle moléculaire et cellulaire. Le rayonnement ultraviolet a donc un fort pouvoir germicide. Ce rayonnement est particulièrement destructeur à la longueur d'onde de 260 nm. Le rayonnement ultraviolet est aussi utilisé à plus forte dose pour la réduction d'ozone et du COT, car il favorise l'oxydation des molécules organiques.

La traitement par rayonnement ultraviolet dans les industries alimentaire, biologique et pharmaceutique est donc intéressant car beaucoup moins énergivore comparé aux traitements thermiques couramment utilisés. Les dispositifs exploitant le rayonnement ultraviolet permettent par ailleurs de proposer des solutions de désinfection sans utilisation de produits chimiques.

On connaît des dispositifs qui utilisent des sources de rayon ultraviolets logées dans des gaines formées de tubes de quartz, de façon à ce que les sources ne soient pas en contact direct avec le fluide à traiter.

L'utilisation de ces tubes de quartz est délicate car ils sont fragiles et peuvent facilement se briser lors d'opérations de maintenance par exemple. Ces opérations de maintenance sont rendues nécessaires, par exemple lorsque le tube s'encrasse, s'oxyde ou s'opacifie.

Le risque de bris du tube de quartz entraîne un risque de contamination du liquide traité par les éclats de ce tube brisé. Ces tubes sont très fragiles lors de toute manipulation, notamment lors du démontage et du nettoyage.

Ainsi le développement des systèmes de traitement, notamment de stérilisation, par rayonnement ultraviolet pour ces industries est freiné par l'emploi de gaines quartz fragiles dans les systèmes conventionnels. Le quartz, naturel ou synthétique, est très transparent dans le domaine de longueur d'onde correspondant aux rayonnement ultraviolet. Cependant, le risque bris de verre/quartz avec des morceaux diffusant dans les réseaux est rédhibitoire dans ces industries.

Par ailleurs, ces tubes ont tendance à subir un encrassement minéral occasionné par les minéraux contenus dans le fluide à traiter, tel que l'eau. Cet encrassement diminue la durée de transmission efficace du flux ultraviolet.

En outre, le quartz, considéré comme dénomination générique de tout matériau à base de silice d'origine minérale naturelle ou de synthèse, offre peu de résistance chimique à certains éléments généralement contenus dans les produits de nettoyage, tels que ceux utilisés notamment dans l'industrie agroalimentaires et pharmaceutiques. Il s'agit par exemple des éléments alcalins forts de type soude. Le nettoyage des gaines est donc problématique. En effet, les systèmes de stérilisation, y compris ceux utilisant des gaines de quartz, sont considérés comme des équipements de procédés et doivent subir à ce titre des cycles de nettoyage visant à nettoyer en ligne (en français : stations de NEttoyage en Place, ou stations NEP; en anglais : Cleaning In Place ou CIP) utilisant de l'acide (acide nitrique, per-acétique) et de la soude ou des solutions alcalines fortes, à 80°C.

En outre, dans certaines applications critiques comme les boucles d'eau ultra pure mises en oeuvre dans l'industrie micro-électronique, le quartz peut se révéler problématique en raison de diffusion de microparticules de SiO₂ dans l'eau, néfaste pour la fabrication des composants électroniques

Un des buts de l'invention est donc de résoudre notamment les problèmes précités. Ainsi, l'invention a notamment pour objectif de proposer un dispositif de traitement d'un fluide, tel que de l'eau, par rayonnement ultraviolet, qui soit notamment peu coûteux, simple à entretenir, efficace et avec une longue durée de vie.

L'invention a ainsi pour objet, un dispositif de traitement d'un fluide, tel que de l'eau ou autre liquide alimentaire ou pharmaceutique, chargés, légèrement opaques, ou encrassant. Le dispositif comprend une chambre pourvue d'une arrivée de fluide à traiter et une sortie de fluide traité. La chambre présente deux extrémités opposées fermées hermétiquement et respectivement par un premier et un deuxième couvercles. Le dispositif comprend une ou plusieurs unités d'émission configurées pour émettre des rayons ultraviolets, UV, à destination d'un fluide présent dans au moins une partie de la chambre, chaque unité d'émission comprenant une gaine rigide et transparente aux rayons UV. La gaine comprend un tube en matériau rigide et transparent aux UV, tel qu'un tube de quartz, dont au moins une partie est logée dans la chambre, le tube comprenant une source d'émission de rayons UV et présentant une section circulaire et une première et une deuxième extrémités ouvertes et opposées. Le dispositif comprend en outre une unité de contrôle électrique configurée pour contrôler le fonctionnement des unités d'émission.

La gaine comprend un fourreau en matériau transparent aux rayons UV, tel que de l'éthylène-propylène fluoré ou du perfluoroalcoxy, plaqué intégralement sur au moins toute la partie du tube (8) logée dans la chambre et dont l'épaisseur est uniformément comprise entre 200 µm et 350 µm, de préférence uniformément comprise entre 200 µm et 300 µm, encore plus préférentiellement uniformément comprise entre 260 µm et 270 µm.

Suivant certains modes de réalisation, le dispositif comprend en outre une ou plusieurs des caractéristiques suivantes, prise(s) isolément ou suivant toutes les combinaisons techniquement possibles :
- le fourreau présente une valeur de rugosité uniformément inférieure ou égale à 0.6 µm, de préférence à inférieure ou égale 0,4 µm, et la gaine rigide présente une valeur de transmittance aux rayons ultraviolets, mesurée pour des rayons ultraviolets de longueur d'onde comprise entre 250 nm et 260 nm, uniformément supérieure ou égale à 45%, de préférence uniformément supérieure ou égale à 48% ;
- le fourreau présente une première et une deuxième extrémités ouvertes et opposées, les premières extrémités respectives du tube et du fourreau formant une première extrémité de la gaine et les deuxièmes extrémités respectives du tube et du fourreau formant une deuxième extrémité de la gaine, la première extrémité de la gaine dépassant de manière étanche du premier couvercle et étant bloquée mécaniquement à travers ce premier couvercle par un premier moyen de blocage configuré pour exercer une compression radiale surfacique sur au moins une partie du périmètre de ladite première extrémité de la gaine, la première extrémité de la gaine étant pourvue d'un connecteur électrique connecté à la source d'émission et destiné à être relié à l'unité de contrôle, la deuxième extrémité de la gaine dépassant de manière étanche du deuxième couvercle et étant bloquée mécaniquement à travers ce deuxième couvercle par un deuxième moyen de blocage configuré pour exercer une compression radiale surfacique sur au moins une partie du périmètre de ladite deuxième extrémité de la gaine ;
- chaque premier et deuxième moyen de blocage comprend un tube lisse avec une première et une deuxième extrémités opposées, et un capuchon, le tube lisse étant encastré et bloqué, par sa première extrémité, dans un trou traversant formé dans le premier, respectivement, deuxième couvercle, la première, respectivement deuxième, extrémité de la gaine traversant le tube lisse depuis la première extrémité dudit tube lisse jusqu'à proximité de la deuxième extrémité dudit tube lisse, le capuchon coopérant avec le tube lisse pour exercer une compression radiale surfacique autour d'au moins une partie du périmètre de la deuxième extrémité du tube lisse, en sorte de bloquer par cette compression radiale surfacique la première, respectivement deuxième, extrémité de la gaine ;
- le dispositif comprend un joint disposé autour de la deuxième extrémité du tube lisse, et en ce que le capuchon coopère avec le tube lisse pour exercer la compression radiale surfacique sur au moins une partie du périmètre de la deuxième extrémité du tube lisse par l'intermédiaire du joint ;
- la première extrémité du tube lisse est fixée sur une surface du premier, respectivement deuxième, couvercle orientée vers l'intérieur de la chambre, par exemple par soudure de cette première extrémité du tube lisse sur ladite surface ;
- le dispositif comprend, pour chaque gaine, un premier bouchon disposé sur le premier couvercle en sorte de couvrir la première extrémité de ladite gaine et le premier moyen de blocage correspondant, ledit premier bouchon portant un élément de connexion électrique traversant de manière étanche ledit premier bouchon, ledit élément de connexion électrique étant d'une part en contact électrique avec le connecteur électrique et d'autre part destiné à être relié à l'unité de contrôle ;

10. Dispositif selon l'une quelconque des revendications 3 à 9, caractérisé en ce qu'il comprend, pour chaque gaine (8, 9), un deuxième bouchon (20) disposé sur le deuxième couvercle (5) en sorte de couvrir la deuxième extrémité de ladite gaine (8, 9) et le deuxième moyen de blocage (13) correspondant.

11. Dispositif selon l'une quelconque des revendications 4 à 6, et 10 caractérisé en ce qu'il comprend un élément de contrôle (21) visuel ou électronique de l'état et du fonctionnement des unités d'émission (6, 7), disposé dans le capuchon (15) de chaque deuxième moyens de blocage (13) ou dans chaque deuxième bouchon (20).

12. Dispositif selon la revendication 11, caractérisé en ce que l'élément de contrôle (21) comprend une paroi permettant un contrôle visuel mais non transparente aux rayons UV, tel qu'une paroi en verre, ou un capteur optique destiné à être relié à l'unité de contrôle (11).

13. Dispositif selon l'une quelconque des revendications 1 à 12, caractérisé en ce que la chambre (1) présente une forme d'ensemble cylindrique, les deux couvercles (4, 5) venant fermer les extrémités respectives de ladite forme d'ensemble sensiblement cylindrique.

L'invention a également pour objet, selon un deuxième aspect, un système de traitement d'un fluide, tel que de l'eau, comprenant une structure rigide sur laquelle est monté un circuit fluidique permettant la circulation d'un fluide depuis une arrivée vers une sortie, ledit circuit fluidique comprenant plusieurs dispositifs de traitement tel que présenté ci-dessus connectés en série ou en parallèle par l'intermédiaire de leurs arrivées et sorties respectives, entre ladite arrivée du circuit fluidique et ladite sortie du circuit fluidique.

Il est précisé que par épaisseur uniformément comprise entre une première et une deuxième valeurs, on entend que l'épaisseur du fourreau est supérieure ou égale à la première valeur et inférieure ou égale à la deuxième valeur tout point de ce fourreau, une fois en position sur le tube.

Par ailleurs, par rugosité du fourreau uniformément inférieure ou égale à une certaine valeur, on entend que la rugosité du fourreau est inférieure ou égale à cette valeur en tout point de la surface externe du fourreau, c'est-à-dire la surface du fourreau qui n'est pas en contact avec la paroi externe du tube, une fois en position sur le tube. Ainsi, l'écart entre l'extrémité haute et la base d'une rugosité présente en un point quelconque de cette surface externe du fourreau est inférieure ou égale à cette valeur.

Également, par valeur de transmittance aux rayons ultraviolets, mesurée pour des rayons ultraviolets de longueur d'onde comprise entre 250nm et 260 nm, uniformément supérieure ou égale à un certain pourcentage, on entend que cette valeur de transmittance est supérieure ou égale à ce pourcentage en tout point de la gaine, comprenant le tube recouvert du fourreau. Ainsi, la quantité de rayonnement ultraviolet, en terme d'énergie, mesurée à l'extérieur de la gaine, est supérieure ou égale à ce pourcentage de la quantité de rayonnement ultraviolet, en terme d'énergie, émise depuis l'intérieur du tube.

Enfin, Il est précisé que par compression radiale surfacique, on entend une compression qui s'exerce radialement en direction de l'axe de la gaine, tout autour de la gaine ou sur au moins une partie du périmètre de celle-ci, et sur une surface de la paroi de la gaine non réduite à une ligne, de préférence s'étendant sur une distance longitudinale supérieure ou égale à 1 cm, en sorte que lorsque l'une des deux extrémités de la gaine est bloquée dans le couvercle correspondant, sans que l'autre des deux extrémités de la gaine soit bloquée dans l'autre couvercle, la gaine est maintenue bloquée dans le couvercle correspondant perpendiculairement à ce dernier.

Ainsi, l'invention exploite le principe du rayonnement ultraviolet pour la désinfection de fluides, en proposant un dispositif compact, facile à coupler à d'autres dispositifs en parallèle ou en série pour augmenter le débit traité et/ou l'efficacité de traitement.

Le dispositif de l'invention permet de traiter un fluide qui le traverse, ou fluide circulant, sans emploi de produit chimique, avec le seul rayonnement ultraviolet produit par la ou les unités d'émission présentes dans la chambre fermée. Le fluide est mis en circulation par un dispositif extérieur tel qu'une pompe, ou par la gravité (écoulement gravitaire).

La présence d'une gaine formée d'un tube à section circulaire, recouvert d'un film en matériau transparent aux UV, de préférence souple, , garantit qu'en cas de bris du tube dans la chambre, aucun éclat de ce tube ne se retrouve dans le liquide.

Les matériaux plastiques fluorés apparaissent comme de bons candidats pour le fourreau, qui doit notamment présenter les caractéristiques suivantes :
- transparent aux rayons UV,
- résistance aux chocs légers,
- capacité à retenir les bris de quartz en cas de casse, impliquant une certaine rigidité et une tenue mécanique, à faible épaisseur,
- résistance chimique (produits de nettoyage, fluides traités) à température pouvant aller jusque 100°C,
- sans diffusion de particules,
- apte au contact alimentaire et pharmaceutique.

Les matériaux de type FEP (éthylène-propylène fluoré) ou PFA (perfluoroalcoxy), sont de bons candidats. Ces matériaux plastiques présentent de très bonnes valeurs de transmittance optique dans le domaine des rayons UV, quoique très inférieures au quartz. Ils sont en revanche très transparents dans le domaine visible.

Aujourd'hui, l'application de revêtement en matériau de type FEP sur des pièces cylindriques est connue. Elle est particulièrement utilisée pour la protection de rouleaux de convoyeurs ou pour protéger des rouleaux d'impression. Elle est utilisée aussi pour protéger des tubes d'éclairage contre les bris de verre en industries alimentaires. Le FEP, matériau très transparent dans le domaine visible est donc particulièrement bien adapté pour cette dernière application. Mais dans ces applications courantes connues, la fonction de protection mécanique est la seule visée. Il en résulte la mise en oeuvre de fourreaux en matériau de type FEP ou PFA thermo-rétractables d'épaisseurs supérieures à 500 µm (voir par exemple WO2010/125389 ou encore US3894236).

Lorsqu'il est question du remplacement de la gaine de quartz par un tube en matériau de type FEP (US3894236), la faible résistance mécanique du matériau limite l'emploi à de faibles diamètres de tubes donc de faibles débits, ou à la multiplication des sections de passages de fluide donc à une complexité des installations, dans le cas où le fluide circule à l'intérieur des tubes.

Lorsqu'il s'agit d'utiliser un fourreau de protection tubulaire entourant une lampe UV avec le fluide circulant autour (WO2010/125389), la tenue mécanique demande une épaisseur de fourreau telle que le rayonnement UV est considérablement atténué, donc insuffisant pour les objectifs de traitement dans les différentes industries précitées.

A titre d'exemple, de tels fourreaux en matériau de type FEP aux épaisseurs standards de 500 µm, thermo-rétracté sur un tube de quartz, présentent une valeur de transmittance finale dans le domaine UV, à une longueur d'onde de 254 nm, inférieure à 40%.

La demanderesse a ainsi déterminé que les caractéristiques d'épaisseur revendiquées pour la gaine permettent d'obtenir la force de rétraction suffisante pour assurer le maintien mécanique des morceaux de quartz en cas de bris du tube, tout en garantissant la transparence requise pour l'efficacité du traitement UV.

En outre, la demanderesse a déterminé que les caractéristiques de rugosité revendiquées permettent de ne pas retenir les impuretés, et donc d'obtenir un niveau d'hygiène suffisant, tel que requis dans la plupart des industries précitées. Elles permettent également d'obtenir un très bon effet non accrochant donc nettoyable.

Le film forme donc un fourreau fin, lisse et uniforme, de faible épaisseur sur tout ou partie de la paroi du tube. Ce fourreau entrave modérément le rayonnement ultraviolet émis par la ou les sources, mais sa faible épaisseur garantit une transmittance globale de la gaine satisfaisante, qui combinée à l'emploi de sources UV de puissance suffisante, garantissent l'obtention des résultats attendu en terme de traitement UV. Il satisfait aux exigences des normes d'hygiène, notamment dans le domaine alimentaire. Il est facilement nettoyable et résiste aux composants chimiques contenus dans les produits nettoyants (y compris la soude). Sa résistance mécanique protège le tube, et assure que le quartz n'est jamais en contact avec le liquide traité même en cas de bris de ce tube.

Il en résulte une augmentation significative de la durée de vie de la gaine.

Le système de maintien du tube recouvert de la gaine, fixé en ses deux extrémités opposées aux couvercles qui ferment la chambre, facilite l'extraction pour le nettoyage et le remplacement du tube, par coulissement du tube une fois débloqués les moyens de blocage correspondant, et après vidange du dispositif. Le système de fixation étanche du tube aux deux extrémités facilite le remplacement de la source d'émission de rayons ultraviolets qui peut aussi être facilement extraite sans retirer le tube lui-même, donc sans vidange.

Ce système de fixation aux extrémités permet le positionnement d'un élément de contrôle visuel de de l'état et du fonctionnement des unités d'émission, totalement hors flux.

Ce système de fixation aux extrémités renforce le maintien mécanique de la gaine, sans qu'il soit nécessaire d'utiliser des moyens complémentaires tels que des moyens de centrage ou autre moyens de fixation à l'intérieur de la chambre. Même dans le cas d'une chambre qui s'étend entre ses deux extrémités sur une distance importante, de tels moyens complémentaires ne sont pas nécessaires. Il en résulte notamment que la chambre est plus facile à nettoyer et qu'elle ne présente aucune zone d'accroche qui favoriseraient la rétention, ce qui est particulièrement important dans les applications de traitement de fluides chargés qui ont tendance à s'accrocher dans de telles zones.

Les caractéristiques et avantages de l'invention apparaîtront à la lecture de la description qui va suivre, donnée uniquement à titre d'exemple, et non limitative, en référence aux figures suivantes :

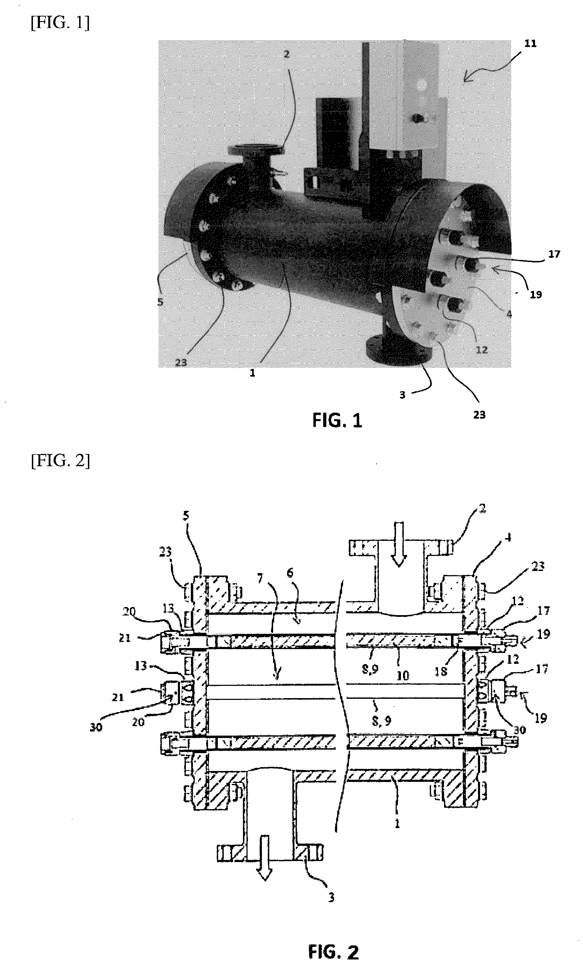

- figure 1 : vue d'ensemble d'un exemple de dispositif selon l'invention ;
- figure 2 : représentation schématique en coupe axiale longitudinale de l'exemple de la figure 1 ;
- figure 3 : représentation schématique d'une des extrémités d'une gaine d'une unité d'émission ;
- figure 4 : représentation schématique en coupe axiale longitudinale d'un des couvercles de l'exemple 3 ;
- figure 5 : vue d'ensemble d'un deuxième moyen de blocage et d'un deuxième bouchon dans l'exemple ;
- figure 6 : représentation schématique en coupe axiale longitudinale du deuxième moyen de blocage et du deuxième bouchon de la figure 6, en position sur un des couvercles ;
- figure 7 : vue d'ensemble d'un premier moyen de blocage et d'un premier bouchon dans l'exemple ;
- figure 8 : représentation schématique en coupe axiale longitudinale du premier moyen de blocage et du premier bouchon de la figure 7, en position sur un des couvercles.

Dans l'exemple représenté sur les figures, le dispositif comprend une chambre 1, de préférence de forme sensiblement cylindrique. La chambre 1 est pourvue d'une arrivée 2 permettant l'arrivée du fluide à traiter, sous pression, ainsi qu'une sortie 3 permettant l'évacuation du fluide une fois traité.

La chambre 1 présente deux extrémités opposées, par exemple dans le cas d'une forme sensiblement cylindrique, les deux extrémité du cylindre en question. Chaque extrémité est fermées hermétiquement par un couvercle 4, 5. Par exemple, des moyens de blocage 23 de type boulon-écrou permettent de plaquer et bloquer hermétiquement chaque couvercle 4, 5 contre des collerettes respectives formées au niveau des deux extrémités de la chambre 1. Le blocage est temporaire, de sorte qu'il est possible de débloquer les couvercles 4, 5, ce qui permet d'inspecter l'intérieur de la chambre 1 et de réaliser des opérations de maintenance et de nettoyage à l'intérieur de cette chambre 1.

Le dispositif comprend une ou plusieurs unités d'émission 6, 7, configurées pour émettre des rayons ultraviolets, UV, à destination du fluide présent dans au moins une partie de cette chambre 1. Dans les deux exemples présentés respectivement sur les figures, la chambre 1 comprend plusieurs unités d'émissions 6, 7.

Chaque unité d'émission 6, 7 comprend une gaine 8, 9 rigide et transparente aux rayons UV. La gaine 8, 9 comprend un tube 8 en matériau rigide et transparent aux UV, tel qu'un tube de quartz. Au moins une partie de ce tube 8 est logée dans la chambre 1.

Chacune des unités d'émission comprend une source d'émission 10 de rayons UV, tel qu'une lampe à amalgame par exemple, logée dans le tube 8.

Une unité de contrôle 11 électrique est prévue, pour contrôler le fonctionnement des unités d'émission 6, 7. L'unité de contrôle 11 peut être intégrée dans un ensemble avec la chambre 1, comme dans l'exemple des figures. Alternativement, l'unité de contrôle peut être distante.

La gaine 8, 9 comprend en outre un fourreau 9 en matériau transparent aux rayons UV, tel que de l'éthylène-propylène fluoré. Ce fourreau 9 est plaqué intégralement sur au moins toute la partie du tube 8 logée dans la chambre 1. Le fourreau 9 présent une première et une deuxième extrémités ouvertes et opposées.

Ce fourreau 9 est uniformément lisse et de faible épaisseur, tout le long de la partie du tube 8 qu'il recouvre. Précisément, la demanderesse a déterminé que les effets recherchés (résistance mécanique, effet d'auto-maintien, éviction des plissures, limitation du risque de fissuration sans détachement de morceaux, bonne transmittance optique dans le domaine UV) étaient obtenus de manière satisfaisante, pour une épaisseur du fourreau 9, en position sur le tube 8, uniformément comprise entre 200 µm et 350 µm, de préférence uniformément comprise entre 200 µm et 300 µm, les meilleurs résultats étant obtenus avec une valeur uniformément comprise entre 260 µm et 270 µm.

De préférence, le fourreau présente, sur toute sa surface, une valeur de rugosité uniformément inférieure ou égale à 0,6 µm, de préférence uniformément inférieure ou égale 0,4 µm. On parle ici de la rugosité mesurée dans le sens longitudinal avec un rugosimètre de contact. Cette faible rugosité rend l'ensemble compatible pour des applications hygiéniques les plus rigoureuses, tel que des application dans le domaine pharmaceutique.

La gaine 8, 9 formée du tube 8 recouvert du fourreau 9 présente une transmittance aux rayons ultraviolets, mesurée pour des rayons ultraviolets de longueur d'onde comprise entre 250nm et 260 nm, uniformément supérieure ou égale à 45%, de préférence uniformément supérieure ou égale à 48%.

Les premières extrémités respectives du tube 8 et du fourreau 9 forment une première extrémité de la gaine 8, 9. Par ailleurs les deuxièmes extrémités respectives du tube 8 et du fourreau 9 forment une deuxième extrémité de la gaine 8, 9.

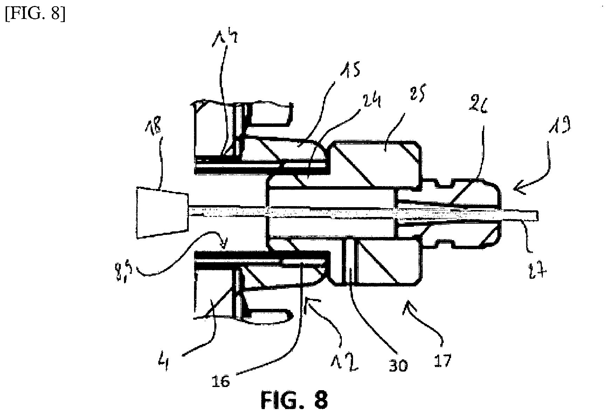

La première extrémité de la gaine 8, 9 dépasse de manière étanche du premier couvercle 4. Elle est bloquée mécaniquement à travers ce premier couvercle 4 par un premier moyen de blocage 12 par compression radiale surfacique exercée sur au moins une partie du périmètre de cette première extrémité de la gaine 8, 9. Un exemple d'un tel premier moyens de blocage 12 est représenté plus en détail sur la figure 8 et sera décrit plus loin.

La première extrémité de la gaine 8, 9 est pourvue d'un connecteur électrique 18 connecté à la source d'émission 10 et destiné à être relié à l'unité de contrôle 11.

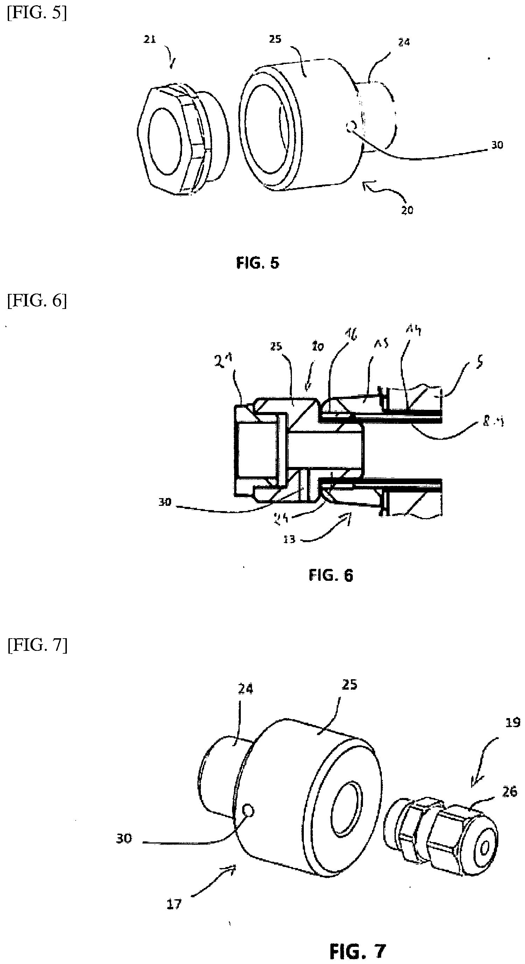

La deuxième extrémité de la gaine 8, 9 dépasse de manière étanche du deuxième couvercle 5 et est bloquée mécaniquement à travers ce deuxième couvercle 5 par un deuxième moyen de blocage 13 par compression radiale surfacique exercée sur au moins une partie du périmètre de cette deuxième extrémité de la gaine 8, 9. Un exemple d'un tel deuxième moyens de blocage 13 est représenté plus en détail sur la figure 6 et est décrit ci-après.

Dans les exemples notamment représentés aux figures 6 et 8, chaque premier et deuxième moyen de blocage 12, 13 comprend un tube lisse 14 et un capuchon 15. Le tube lisse 14 présente une première et une deuxième extrémités opposées. Il est encastré et bloqué, par sa première extrémité, dans un trou traversant formé dans le premier, couvercle 4 en ce qui concerne le premier moyen de blocage 12, ou dans le deuxième couvercle 5 en ce qui concerne le deuxième moyen de blocage 13.

La première extrémité de la gaine 8, 9 traverse le tube lisse 14 du premier moyen de blocage 12, depuis la première extrémité de ce tube lisse 14 jusqu'à proximité de la deuxième extrémité de ce tube lisse 14. Par ailleurs, la première extrémité de la gaine 8, 9 traverse le tube lisse 14 du deuxième moyen de blocage 13, depuis la première extrémité de ce tube lisse 14 jusqu'à proximité de la deuxième extrémité de ce tube lisse 14.

Le capuchon 15 du premier moyen de blocage 12 est monté pour exercer une compression radiale surfacique autour de la deuxième extrémité du tube lisse 14, en sorte de bloquer par compression radiale surfacique la première extrémité de la gaine 8, 9. De même, le capuchon 15 du deuxième moyen de blocage 13 est monté pour exercer une compression radiale surfacique autour de la deuxième extrémité du tube lisse 14, en sorte de bloquer par compression radiale surfacique la deuxième extrémité de la gaine 8, 9.

Dans l'exemple représenté, un joint 16 est disposé autour de la deuxième extrémité du tube lisse 14, entre le capuchon 15 et cette deuxième extrémité du tube lisse 14. Il s'agit d'un joint large, présentant une surface de contact autour du tube lisse 14 et une surface de contact opposée à l'intérieur du capuchon 15, ces surfaces de contact étant non réduites à une ligne de contact. La distance ou dimension longitudinale de ces surfaces de contact est configurée pour assurer la compression radiale surfacique. Par exemple une surface de contact de dimension ou distance longitudinale supérieure ou égale à 1 cm permet d'obtenir le blocage souhaité.

Le capuchon 15 exerce alors une compression radiale surfacique autour de la deuxième extrémité du tube lisse 14 par l'intermédiaire de ce joint 16. Le capuchon 15 est par exemple vissé, par l'intermédiaire d'un taraudage, sur un filetage présent sur la deuxième extrémité du tube lisse 14.

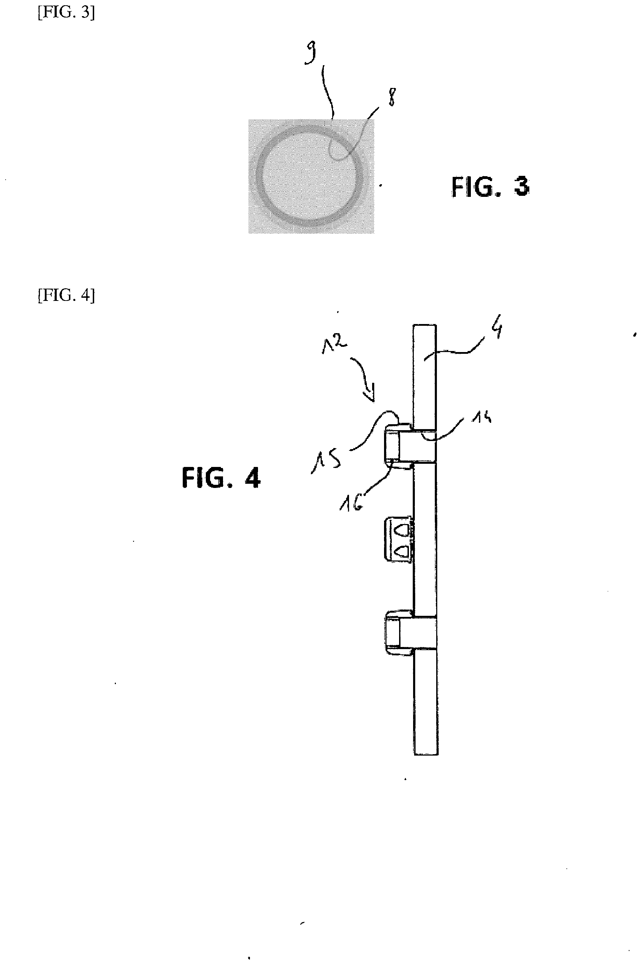

Comme on peut le voir plus précisément sur la figure 4 dans l'exemple des premiers moyens de blocage 12 montés dans le premier couvercle 4, la première extrémité du tube lisse 14 est fixée sur une surface de ce premier couvercle 4, orientée vers l'intérieur de la chambre 1. Cette fixation peut par exemple être obtenue par soudure de cette première extrémité du tube lisse 14 sur la surface en question du premier couvercle 4.

Pour faciliter la soudure, la première extrémité du tube 14 peut être chanfreinée, tout comme l'arrête intérieure du trou traversant le premier couvercle 4 et dans lequel s'insère le tube lisse 14. On peut donc réaliser une soudure par cordon intérieur poli et lissé, de préférence également passivé. Une fois cette soudure réalisée pour chaque moyen de blocage 12, la surface intérieure du premier couvercle 4 est lisse et sans aspérité.

Les mêmes considérations s'applique pour le montage des deuxièmes moyens de blocage 13 dans le deuxième couvercle 5.

Ainsi, les premiers moyens de blocage 12 et les deuxièmes moyens de blocage 13 permettent le passage et le blocage radial, de manière étanche, respectivement de la premières extrémité et de la deuxième extrémité de la gaine 8, 9, au travers respectivement du premier couvercle 4 et du deuxième couvercle 5, par un moyen de type presse-étoupe.

Chaque gaine 8, 9 est associée à un premier bouchon 17 distinct, disposé sur le premier couvercle 4 en sorte de couvrir la première extrémité de la gaine 8, 9 et le premier moyen de blocage 12 correspondant. Chacun de ces premiers bouchon 17 porte alors un connecteur électrique 19 traversant de manière étanche ce premier bouchon 17. Là encore, cet élément de connexion électrique 19 est d'une part en contact électrique avec le connecteur électrique 18 et d'autre part destiné à être relié à l'unité de contrôle 11.

Comme on peut le voir sur la figure 8, l'élément de connexion 19 comprend un fil 27 relié au connecteur électrique 18, traversant de manière étanche le premier bouchon 17 au moyen d'un élément de passage étanche 26, par exemple de type presse-étoupe.

On peut voir sur les figures 7 et 8 que le premier bouchon 17 comprend une partie 24 qui s'encastre dans une ouverture formée dans le capuchon 15 du premier moyen de blocage 12, surmontée d'une partie 25 dans laquelle est disposée l'élément de connexion électrique 19 par l'intermédiaire de son élément de passage étanche 26.

Dans l'exemple représenté sur les figures, chaque gaine 8, 9 est associée à un deuxième bouchon 20, disposé sur le deuxième couvercle 5 en sorte de couvrir la deuxième extrémité de ladite gaine 8, 9 et le deuxième moyen de blocage 13 correspondant.

On peut voir sur les figures 5 et 6 que le deuxième bouchon 20 comprend une partie 24 qui s'encastre dans une ouverture formée dans le capuchon 15 du deuxième moyen de blocage 13, surmontée d'une partie 25.

Dans le cas particulier représenté sur les figures, la partie 25 est fermée par un élément de contrôle 21 visuel de l'état et du fonctionnement des unités d'émission 6, 7. Il peut s'agir d'une paroi permettant un contrôle visuel, mais non transparente aux rayons UV, tel qu'une paroi en verre.

Alternativement, la partie 25 peut être fermée par un élément de contrôle électronique de l'état et du fonctionnement des unités d'émission 6, 7. Il peut s'agir capteur optique destiné à être relié à l'unité de contrôle 11, permettant ainsi un contrôle automatique.

L'élément de contrôle visuel ou électronique pourrait être également disposé dans l'ouverture formée dans le capuchon 15 de chaque deuxième moyen de blocage 13, dans le cas où les deuxièmes bouchons 20 ne sont pas présent. L'élément de contrôle visuel ou électronique vient alors fermer le capuchon 15. Si les deuxièmes bouchons 20 sont présent, l'élément de contrôle visuel ou électronique peut également être disposé dans l'ouverture formée dans le capuchon 15 de chaque deuxième moyen de blocage 13, mais le matériau des deuxièmes bouchons 20 doit être suffisamment transparent pour permettre le contrôle visuel.

Les premiers et deuxièmes moyens de blocage 12, 13 et/ou les premiers et deuxièmes bouchons 17, 20 comprennent des moyens de décompression, configurés pour permettre l'évacuation de l'air chaud contenu dans la gaine 8, 9. Cet air chaud provient de l'augmentation de température provoquée par le fonctionnement des sources d'émission UV 10.

Dans les exemples représentés sur les figures, en particulier sur les figures 5 à 8, ces moyens de décompression prennent la forme d'une cheminée 30 formée dans les premiers et deuxièmes bouchons 17, 20. Dans ces exemples, cette cheminée 30 est formée dans la partie 25 de ces bouchons 17, 20. D'autres configurations et positionnement de la cheminée 30 sont possibles, par exemple dans l'une ou l'autre des parties 24, 25 des bouchons 17, 20, et/ou dans les premiers et deuxièmes moyens de blocage 12, 13, plus généralement en fonction de la forme de ces bouchons 17, 20 et de ces premiers et deuxièmes moyens de blocage 12, 13, et de leurs agencement relatif, pour autant que ces autres configurations permettent l'évacuation de l'air chaud depuis l'intérieur de la gaine vers l'extérieur du dispositif.

Pour assurer une capacité de traitement importante, on prévoit de connecter plusieurs dispositifs tels que présentés ci-dessus, au sein d'un système de traitement d'un fluide, tel que de l'eau.

Plus généralement, ce système comprend une structure rigide, sur laquelle est monté un circuit fluidique permettant la circulation d'un fluide sous pression depuis une arrivée. Les dispositifs de traitement sont connectés en série ou en parallèle par leurs arrivées et sorties respectives, entre l'arrivée du circuit fluidique et la sortie du circuit fluidique.

Ainsi, le dispositif de l'invention permet une stérilisation UV, avec un fort débit. Il est facilement nettoyable. Le matériau rigide formant le tube 8, tel que du quartz, n'est jamais en contact avec le liquide traité. Le fourreau 9 recouvrant le tube 8 présente une résistance chimique et mécanique importante.

Les propriétés d'anti-adhérence du matériau de la gaine recouvrant chaque tube, tel que de l'éthylène-propylène fluoré, le rende très lisse et non accrochant. Ainsi le fourreau 9 rend le dispositif adapté au traitement de liquides légèrement chargés ou accrochant.

Ce fourreau 9 se nettoie donc facilement grâce notamment à ses propriétés lisses et non accrochantes. Associé à une chambre 1, par exemple en plastique lisse type PEHD résistant aux rayons UV ou en inox de qualité alimentaire ou pharmaceutique, le dispositif est complètement lisse, pour sa partie au contact du liquide ou fluide à traiter, sans zone de rétention, et parfaitement nettoyable.

Un flux de liquide caractérisé par un nombre de Reynolds supérieur à 10000 permet un écoulement turbulent et autonettoyant, dont l'effet renforcé par les propriétés de glissement à la surface du fourreau 9.

Comme déjà évoqué, le fourreau 9 renforce significativement la résistance mécanique de la gaine qui entoure la source d'émission UV. L'ensemble tolère des chocs légers. En cas de choc fort, le tube 8 conserve son intégrité. Lorsque le fourreau 9 exerce des contraintes compressives suffisantes vers l'intérieur du tube 8, en cas de choc, le tube 8 se fissure sans séparation des morceaux, à la manière du feuilletage d'un pare-brise de voiture.

Le système de fixation, traversant, par encastrement type presse-étoupe avec joints larges et lisses, aux deux extrémités de la chambre 1, permet d'empêcher tout passage de bris de tube 8 dans le liquide traité, en cas de casse de ce tube. En cas de choc fort au milieu de la gaine 8, 9 pouvant rompre la tube 8, les morceaux du tube 8 restent dans le fourreau 9, le maintien rigide étant assuré aux extrémités au niveau des moyens de blocage 12, 13 dans les couvercles 4, 5. L'évacuation des morceaux éventuels peut se faire après extraction des sources UV 10 (sans outil) et retrait des capuchons 15 des moyens de blocage 12, 13. Le fourreau 9 est ainsi maintenu aux extrémités, en l'on peut faire glisser le tube cassé en la maintenant horizontal, à l'aide par exemple d'une tige revêtue de PTFE traversant la gaine 8, 9 par son axe central.

Les fixations étant disposées aux extrémités de la chambre 1, il n'existe aucun élément à l'intérieur de cette chambre 1 susceptible de nuire au nettoyage ou de subir un encrassage. L'écoulement interne, dans la chambre cylindrique 1, est de type parfaitement annulaire autour des gaines 8, 9.

Le fourreau 9, par exemple en matériau de type FEP ou PFA, est appliqué par thermo-rétraction, en sorte de présenter l'épaisseur résiduelle finale recherchée. Comme évoqué plus haut, celle-ci est uniformément comprise entre 200µm et 350µm, de préférence comprise entre 200 µm et 300 µm, encore plus préférentiellement comprise entre 260 µm et 270 µm. La gaine 8, 9 ainsi obtenue est résistante aux chocs légers. En cas de bris du tube 8, le revêtement par le fourreau 9 agit comme un feuilletage sur pare-brise, le tube 8 se fissurant mais avec les morceaux encore auto maintenus par la force de rétraction du fourreau 9

Avec entre 200 µm et 250 µm d'épaisseur, la résistance mécanique et l'effet d'auto-maintien sont encore suffisants, bien que considérablement atténués. La transmittance optique dans le domaine UV à 254nm est satisfaisante (supérieure à 58%). Mais l'application du fourreau 9 devient délicate et le risque de plissures sur ce fourreau 9 est non négligeable. La demanderesse a déterminé qu'en dessous de l'épaisseur de 200 µm après rétreint, la résistance mécanique, l'effet d'auro-maintien, et l'éviction des plissures, n'étaient plus suffisants. Ceci est en particulier le cas pour un tube 8 de quartz présentant un ratio longueur/diamètre supérieur à 42. En outre, en dessous de cette valeur d'épaisseur, le risque de fissuration sans détachement de morceaux est important

Par ailleurs, au-delà de 300 µm d'épaisseur, l'effet de protection mécanique est très bon. La gaine est résistante à un choc de force 0,200 J. Avec un telle valeur d'épaisseur, en cas de choc, les morceaux sont contenus dans le fourreau 9, sans perforation de celui-ci. L'aspect très lisse du fourreau 9 après application, sans aucune plissure, est obtenu. La transmittance aux rayons UV à 254nm, de l'ensemble tube 8 plus fourreau 9, reste supérieure ou égale à 45% (de l'ordre de 48%), ce qui constitue la limite acceptable pour une bonne efficacité UV sans multiplier outre mesure le nombre de lampes. Au-delà de l'épaisseur de 350 µm pour le fourreau 9, la transmittance tombe en dessous de 48%, ce qui n'est plus acceptable.

L'invention, avec les caractéristiques déterminées par la demanderesse, et telles que revendiquées, constitue une solution efficace, à coût modéré, pour des systèmes de traitement UV appliqués notamment à :
- la pasteurisation à froid de liquides des industries alimentaires, biologiques et pharmaceutiques,
- au traitement de stérilisation d'eau avec impuretés,
- aux procédés de traitement d'effluents par oxydation avancée.

L'association avec des lampes à haute émission UV de l'invention, dans le même encombrement que les lampes classiques telles que les lampes à amalgame, permet de compenser la légère baisse de transmittance du système optique et de concevoir ainsi un système UV avec le même nombre de lampes que les systèmes UV conventionnels.

On rappelle que la présente description est donnée à titre d'exemple et n'est pas limitative de l'invention. En particulier, l'invention ne se limite pas à un dispositif dont la chambre de traitement présente une forme spécifique. De même l'invention ne se limite pas à un dispositif présentant un nombre déterminé d'unité d'émission de rayons UV.

Par ailleurs, l'invention ne se limite pas non plus à l'utilisation d'une source particulière de rayons UV.

## Revendications

1. Dispositif de traitement d'un fluide, tel que de l'eau, comprenant une chambre (1) pourvue d'une arrivée (2) de fluide à traiter et une sortie (3) de fluide traité, la chambre (1) présentant deux extrémités opposées fermées hermétiquement et respectivement par un premier et un deuxième couvercles (4, 5), le dispositif comprenant une ou plusieurs unités d'émission (6, 7) configurées pour émettre des rayons ultraviolets, UV, à destination d'un fluide présent dans au moins une partie de la chambre (1), chaque unité d'émission (6, 7) comprenant une gaine (8, 9) rigide et transparente aux rayons UV, la gaine (8, 9) comprenant un tube (8) en matériau rigide et transparent aux UV, tel qu'un tube de quartz, dont au moins une partie est logée dans la chambre (1), le tube (8) comprenant une source d'émission (10) de rayons UV et présentant une section circulaire et une première et une deuxième extrémités ouvertes et opposées, le dispositif comprenant en outre une unité de contrôle (11) électrique configurée pour contrôler le fonctionnement des unités d'émission (6, 7), la gaine (8, 9) comprenant un fourreau (9) en matériau transparent aux rayons UV, tel que de l'éthylène-propylène fluoré ou du perfluoroalcoxy, plaqué intégralement sur au moins toute la partie du tube (8) logée dans la chambre (1) et dont l'épaisseur est uniformément comprise entre 200 µm et 350 µm, de préférence comprise entre 200 µm et 300 µm, encore plus préférentiellement comprise entre 260 µm et 270 µm.

2. Dispositif selon la revendication 1, **caractérisé en ce que** le fourreau (9) présente une valeur de rugosité uniformément inférieure ou égale à 0.6 µm, de préférence à inférieure ou égale 0,4 µm, et **en ce que** la gaine rigide (8, 9) présente une valeur de transmittance aux rayons ultraviolets, mesurée pour des rayons ultraviolets de longueur d'onde comprise entre 250 nm et 260 nm, uniformément supérieure ou égale à 45%, de préférence uniformément supérieure ou égale à 48%.

3. Dispositif selon l'une quelconque des revendications 1 et 2, **caractérisé en ce que** le fourreau (9) présente une première et une deuxième extrémités ouvertes et opposées, les premières extrémités respectives du tube (8) et du fourreau (9) formant une première extrémité de la gaine (8, 9) et les deuxièmes extrémités respectives du tube (8) et du fourreau (9) formant une deuxième extrémité de la gaine (8, 9), la première extrémité de la gaine (8, 9) dépassant de manière étanche du premier couvercle (4) et étant bloquée mécaniquement à travers ce premier couvercle (4) par un premier moyen de blocage (12) configuré pour exercer une compression radiale surfacique sur au moins une partie du périmètre de ladite première extrémité de la gaine (8, 9), la première extrémité de la gaine (8, 9) étant pourvue d'un connecteur électrique (18) connecté à la source d'émission (10) et destiné à être relié à l'unité de contrôle (11), la deuxième extrémité de la gaine (8, 9) dépassant de manière étanche du deuxième couvercle (5) et étant bloquée mécaniquement à travers ce deuxième couvercle (5) par un deuxième moyen de blocage (13) configuré pour exercer une compression radiale surfacique sur au moins une partie du périmètre de ladite deuxième extrémité de la gaine (8, 9).

4. Dispositif selon la revendication 3, **caractérisé en ce que** chaque premier et deuxième moyen de blocage (12, 13) comprend un tube lisse (14) avec une première et une deuxième extrémités opposées, et un capuchon (15), le tube lisse (14) étant encastré et bloqué, par sa première extrémité, dans un trou traversant formé dans le premier, respectivement, deuxième couvercle (4, 5), la première, respectivement deuxième, extrémité de la gaine (8, 9) traversant le tube lisse (14) depuis la première extrémité dudit tube lisse (14) jusqu'à proximité de la deuxième extrémité dudit tube lisse (14), le capuchon (15) coopérant avec le tube lisse (14) pour exercer une compression radiale surfacique autour d'au moins une partie du périmètre de la deuxième extrémité du tube lisse (14), en sorte de bloquer par cette compression radiale surfacique la première, respectivement deuxième, extrémité de la gaine (8, 9).

5. Dispositif selon la revendication 4, **caractérisé en ce qu'**il comprend un joint (16) disposé autour de la deuxième extrémité du tube lisse (14), et **en ce que** le capuchon (15) coopère avec le tube lisse (14) pour exercer la compression radiale surfacique sur au moins une partie du périmètre de la deuxième extrémité du tube lisse (14) par l'intermédiaire du joint (16).

6. Dispositif selon l'une quelconque des revendications 4 et 5, **caractérisé en ce que** la première extrémité du tube lisse (14) est fixée sur une surface du premier, respectivement deuxième, couvercle (4, 5) orientée vers l'intérieur de la chambre (1), par exemple par soudure de cette première extrémité du tube lisse (14) sur ladite surface.

7. Dispositif selon l'une quelconque des revendications 3 à 6, **caractérisé en ce qu'**il comprend, pour chaque gaine (8, 9), un premier bouchon (17) disposé sur le premier couvercle (4) en sorte de couvrir la première extrémité de ladite gaine (8, 9) et le premier moyen de blocage (12) correspondant, ledit premier bouchon (17) portant un élément de connexion électrique (19) traversant de manière étanche ledit premier bouchon (17), ledit élément de connexion électrique (19) étant d'une part en contact électrique avec le connecteur électrique (18) et d'autre part destiné à être relié à l'unité de contrôle (11).

8. Dispositif selon l'une quelconque des revendications 3 à 7, **caractérisé en ce qu'**il comprend, pour chaque gaine (8, 9), un deuxième bouchon (20) disposé sur le deuxième couvercle (5) en sorte de couvrir la deuxième extrémité de ladite gaine (8, 9) et le deuxième moyen de blocage (13) correspondant.

9. Dispositif selon l'une quelconque des revendications 4 à 5, et 8 **caractérisé en ce qu'**il comprend un élément de contrôle (21) visuel ou électronique de l'état et du fonctionnement des unités d'émission (6, 7), disposé dans le capuchon (15) de chaque deuxième moyens de blocage (13) ou dans chaque deuxième bouchon (20).

10. Dispositif selon la revendication 11, **caractérisé en ce que** l'élément de contrôle (21) comprend une paroi permettant un contrôle visuel mais non transparente aux rayons UV, tel qu'une paroi en verre, ou un capteur optique destiné à être relié à l'unité de contrôle (11).

11. Dispositif selon l'une quelconque des revendications 1 à 10, **caractérisé en ce que** la chambre (1) présente une forme d'ensemble cylindrique, les deux couvercles (4, 5) venant fermer les extrémités respectives de ladite forme d'ensemble sensiblement cylindrique.

12. Système de traitement d'un fluide, tel que de l'eau, comprenant une structure rigide sur laquelle est monté un circuit fluidique permettant la circulation d'un fluide depuis une arrivée (31) vers une sortie (22), ledit circuit fluidique comprenant plusieurs dispositifs (28, 29, 32) de traitement selon l'une quelconque des revendications 1 à 11 connectés en série ou en parallèle par l'intermédiaire de leurs arrivées (2) et sorties (3) respectives, entre ladite arrivée (31) du circuit fluidique et ladite sortie (22) du circuit fluidique.
